(19) Europäisches Patentamt
European Patent Office
Office européen des brevets

(11) **EP 2 314 237 A1**

(12) **EUROPEAN PATENT APPLICATION**

(43) Date of publication:
**27.04.2011 Bulletin 2011/17**

(51) Int Cl.:
***A61B 17/54*** *(2006.01)*

(21) Application number: **10196030.0**

(22) Date of filing: **22.06.2006**

(84) Designated Contracting States:
**DE FR**

(30) Priority: **23.06.2005 US 693139 P**
**15.06.2006 US 453485**

(62) Document number(s) of the earlier application(s) in accordance with Art. 76 EPC:
**06773850.0 / 1 893 108**

(71) Applicant: **Johnson & Johnson Consumer Companies, Inc.**
**Skillman, NJ 08558 (US)**

(72) Inventors:
• **Menke, James C.**
**Califon, NJ 07830 (US)**
• **Eknoian, Michael**
**Warren, NJ 07059 (US)**
• **Hull, Raymond J.**
**Hampton, NJ 08827 (US)**

• **Cole, Curtis**
**Ringoes, NJ 08551 (US)**
• **Gubernick, David**
**Cherry Hill, NJ 08003 (US)**
• **Lubrizzi, Joseph J.**
**Hillsborough, NJ 08844 (US)**
• **Luizzi, Joseph, M**
**Newtown, PA 18940 (US)**
• **McLaughlin, Robert**
**Milltown, NJ 08850 (US)**

(74) Representative: **Kirsch, Susan Edith**
**Carpmaels & Ransford**
**One Southampton Row**
**London**
**WC1B 5HA (GB)**

Remarks:
This application was filed on 20-12-2010 as a divisional application to the application mentioned under INID code 62.

(54) **Mechanical skin resurfacing**

(57)     An article useful for mechanical skin resurfacing techniques is suitable for transferring mechanical energy from a handheld device to skin placed in contact with the article. The article may be characterized by its Durable Abrasiveness, Compressibility, Displacement, and/or surface roughness. The article may be formed of a fibrous structure having a first major surface having associated therewith an adhesive system and a second major surface, generally opposite the first major surface. The second major surface is arranged and configured to reversibly engage a fastener of a motion-generating unit. The invention also relates to a coupling device for coupling a motorized apparatus to a disposable skin-contactable element. The coupling device includes a water-resistant first attachment and a second attachment for releasably affixing said article to a surface of the motion generation unit.

FIG. 1

EP 2 314 237 A1

**Description**

BACKGROUND OF THE INVENTION

With advances in nutrition and medical treatment, the life expectancy of the average U.S. and world citizen has increased dramatically. As a result, large portions of those populations suffer from the associated effects of aging, including an increasing number of skin health issues. Though seldom life threatening, skin health issues can be uncomfortable and may cause chronic disabilities. In addition, because the skin is so visible, skin health issues and cosmetic skin conditions can lead to psychological stress in the patients who have them. These factors have driven people to seek improved solutions to health care and skin care.

Numerous techniques have been proposed to provide cosmetic and/or or skin rejuvenation benefits. One of the more popular techniques, professional microdermabrasion, is a non-invasive procedure in which a device pulls the skin via suction and bombards the skin with abrasive particles in order to affect an exfoliation. Professional microdermabrasion devices, however, are cumbersome in that they occupy a large amount of space and also require a high power input and must be plugged into an AC outlet during operation. Furthermore, the patient must make regular visits to the professional skin care specialist where he or she receives treatment. Accordingly, "at home" microdermabrasion systems that combine a motorized apparatus and an abrasive system are now available.

Applicants have recognized that while "at-home" microdermabrasion systems are commercially available, these systems, while efficacious, may be less than optimal for various reasons. Available systems often employ an abrasive cream that may be costly and require effort to rinse from the skin. Other systems may employ a surface for contacting the skin that is an integral part of a twit or module that requires periodic replacement. The module may be often costly to manufacture, and therefore, costly to replace. Other systems employ a skin-contacting surface that can be attached to a device via a "peel and stick" type adhesive. Unfortunately, these adhesives are prone to failure during use, in that the adhesive may lose its grip due to water that is generally present on the skin or used with the apparatus during treatment. Yet other systems have attempted to simply provide a motorized platform to use commercially available skin cleansing pads without providing significant abrasive action to the skin. As such, it may be desirable to couple the microdermabrasion tool to a medium such as a carrier or pad to provide a pleasant and efficacious and cost-effective skin treatment, especially to a medium that is capable of transferring the mechanical action from the motorized device to the skin surface. Accordingly, a need exists for a systems, articles, and methods and compositions that overcome one or more of the above-mentioned drawbacks.

SUMMARY OF THE INVENTION

In one aspect, embodiments of the invention relate to an article useful for mechanical skin resurfacing techniques. In a first embodiment, the article is suitable for transferring mechanical energy from a handheld device to skin placed in contact with the article, wherein the article has a Durable Abrasiveness from 2 to 14.

In another embodiment, the article is suitable for transferring mechanical energy from a handheld device to skin placed in contact with the article, and the article has a Durable Abrasiveness greater than 1, but less than about 14, and a Compressibility from about 7% to about 18%.

In another embodiment, the article is suitable for transferring mechanical energy from a handheld device to skin placed in contact with the article, and the article has a Durable Abrasiveness greater than 1, but less than about 14, and a Displacement from about 0.15mm to about 2mm.

In another embodiment, the article is suitable for transferring mechanical energy from a handheld device to skin placed in contact with the article, and the article has a Displacement from about 0.15 mm to about 2.0mm, preferably from about 0.25mm to about 1mm, more preferably from about 0.25 to about 0.8mm, and most preferably from about 0.25mm to about 0.5mm; and the article has a maximum surface roughness from about 200 microns to about 3000 microns, preferably from about 300 microns to about 2000 microns, more preferably from about 350 microns to about 1500 microns, and even more preferably from about 400 microns to about 1200 microns.

In another aspect of the invention, a method of treating an expanse of skin includes imparting to an expanse of skin, mechanical energy via an apparatus comprising (1) a motor and (2) a skin-contactable element described in this Summary of the Invention; and contacting said expanse of skin with the skin-contactable element.

Another embodiment of the invention includes a skin-contactable element including a fibrous structure and an abrasive system bound to such structure. The abrasive system may be chemically bonded (including adhesively bonded) to the fibers.

In another embodiment, a disposable skin treatment element is formed of a fibrous structure having a first major surface having associated therewith an adhesive system and a second major surface, generally opposite the first major surface. The second major surface is arranged and configured to reversibly engage a fastener of a motion-generating unit.

In another embodiment, a skin-contactable element may be formed of a network of fibers with a plurality of discrete abrasive units bound to said fibers, wherein said discrete abrasive units comprise a polymer having a glass transition temperature greater than about -20°C, wherein portions of said fibers and portions of said discrete abrasive units form a skin-contactable surface.

In another embodiment, a system for mechanical skin resurfacing techniques includes an apparatus that has a loop-engageable surface for reversibly engaging a fibrous skin-contactable element thereto coupled with a motion-generating unit. The loop-engageable surface may include a plurality of protrusions for engaging said fibrous skin-contactable element.

In yet another embodiment, a coupling device for coupling a motorized apparatus to a disposable skin-contactable element includes a water-resistant first attachment and a second attachment for releasably affixing said article to a surface of the motion generation unit. The first attachment is useful to releasably attach the skin-contactable element to said coupling article, and the first and said second attachments have sufficient strength to substantially maintain a position of the skin-contactable element relative to the surface of the apparatus when the skin-contactable is urged against the skin.

In an alternative embodiment, a system for mechanical skin resurfacing techniques includes a motion generation unit, a disposable, skin-contactable element; and an adaptor comprising a water-resistant first attachment for releasably affixing the skin-contactable, fibrous pad to said adaptor and a second attachment for releasably affixing the handheld, motorized apparatus to said adaptor.

BRIEF DESCRIPTION OF THE DRAWINGS

A more particular description of the invention, briefly summarized above may be had by reference to the embodiments thereof that are illustrated in the appended drawings. It is to be so noted, however, that the appended drawings illustrate only typical embodiments of the invention and, therefore, are not to be considered limiting of its scope, for the invention may admit to other equally effective embodiments.

Figure 1 is a schematic side view of a system for treating the skin that is consistent with embodiments of the invention described herein;

Figure 2 is a fragmented, schematic side view of an apparatus and a skin-contactable element having a loop-engageable surface, consistent with embodiments of the invention described herein;

Figure 3A is a cross-sectional view of the loop-engageable surface of Figure 2, showing protrusions thereon;

Figure 3B is another embodiment of the loop-engageable surface of Figure 2;

Figure 4A is a cross-sectional view of a skin-contactable element consistent with embodiments of the invention described herein;

Figure 4B is a top view of the skin-contactable element of Figure 4A;

Figure 5 is a cross-sectional view of a skin-coiitactable element consistent with embodiments of the invention described herein;

Figure 6 is a schematic side view of a system for treating the skin, said system including an adaptor, consistent with embodiments of the invention described herein; and

Figure 7 is a perspective view of the adaptor of Figure 6. To facilitate understanding identical reference elements have been used, wherever possible, to designate identical elements that are common to the figures.

Figures 8-14 show perspective view of different embodiments of the adapter of Figure 6.

DETAILED DESCRIPTION OF THE INVENTION

It is believed that one skilled in the art can, based upon the description herein, utilize the present invention to its fullest extent. The following specific embodiments are to be construed as merely illustrative, and not limitative of the remainder of the disclosure in any way whatsoever.

It is believed that one skilled in the art can, based upon the description herein, utilize the present invention to its fullest extent. The following specific embodiments are to be construed as merely illustrative and not limiting the remainder of the disclosure in any way whatsoever.

As used herein the specification and the claims, the term "mechanical skin resurfacing technique" and variants thereof relate to the mechanically assisted removal of mammalian (especially human) skin cells, ranging from mild techniques (such as exfoliation and abrasive cleansing) through mícrodermabrasion, and up to severe techniques such as dermal abrasion.

As used herein the specification and the claims, the term "dermabrasion" and variants thereof relate to a non-thermal resurfacing technique especially well suited for deep defects of the skin such as acne scars, heavy wrinkles and the disfiguring effects of skin conditions like rosacea. The procedure involves the mechanical sanding of the upper layers of the skin and penetrates the skin deeper than microdermabrasion. With dermabrasion, a new layer of skin replaces the abraded skin during healing, resulting in a smoother appearance

As used herein the specification and the claims, the term "microdermabrasion" and variants thereof relate to a very mild and less-penetrating form of dermabrasion, more suited for reduction of fine lines and wrinkles and for other less severe skin conditions. Microdermabrasion penetrates less deeply into the skin, primarily the stratum corneum, or portions thereof.

As used herein the specification and the claims, the term "exfoliation"and variants thereof relate to the peeling and sloughing off of the skin's tissue cells.

As used herein the specification and the claims, the term "cleansing" and variants thereof relate to removal of dirt, oils, and the like from the surface of the skin, especially through surfactant washing, and perhaps also penetrating into the pores of the skin. In "abrasive cleansing," some degree of exfoliation also occurs.

These mechanical skin treatments may facilitate the delivery of benefit agents to skin tissue, e.g., cleansing and the delivery of acne treatment compositions or rejuvenating agents such as retinol.

As used herein the specification and the claims, the term "nonwoven" and variants thereof relate to a sheet, web, or bat of natural and/or man-made fibers or filaments, excluding paper, that have not been converted into yarns, and that are bonded to each other by any of several means. For additional clarification, nonwovens are distinct from woven and knitted fabrics. The fibers included in the nonwoven materials may be staple or continuous or be formed in situ, and preferably, at least about 50% of the fibrous mass is provided by fibers having a length to diameter ratio greater than about 300:1.

The present invention is directed to systems, articles, compositions, and methods useful for mechanical skin resurfacing techniques employing a handheld motorized device. In various embodiments of the invention, such systems, articles, and methods provide a unique combination of high reliability and convenience for the user, as well as a highly efficacious mechanical skin resurfacing technique.

Systems useful for mechanical skin resurfacing techniques according to embodiments of the present invention may vary with respect to presence or absence of various components or sub-assemblies; the size, shape, and selection of materials, and the like. For a description of various systems for treating the skin and various portions of said systems, the reader is referred to co-pending published patent application, US2005-0148907, filed December 24, 2003, entitled "TREATMENT OF SKIN USING A BENEFIT AGENT AND AN APPARATUS," herein incorporated by reference. Of particular note are those sections entitled, "SKIN TREATMENT SYSTEMS "MECHANICAL ENERGY DELIVERY SUB-ASSEMBLY," "ACTUATION OF SKIN-CONTACTABLE SURFACE," "CHEMICAL DELIVERY SUB-ASSEMBLY," "INDICATOR," "CONTROLLER," "RECEIVING ELEMENT AND SENSING ELEMENT," "WAVEFORM CONTROL," "BENEFIT AGENTS," "DIAGNOSTIC SUB-SYSTEM," AND "METHOD OF USE."

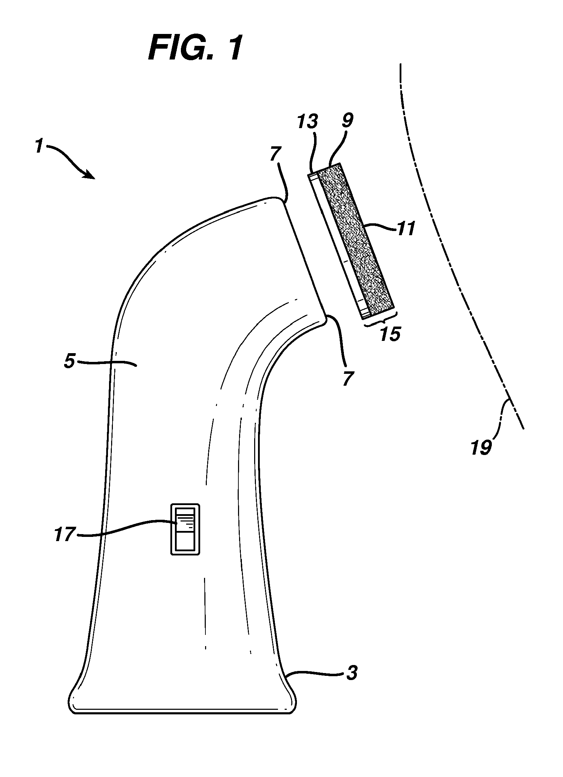

Figure 1 depicts one non-limiting example of a system 1 useful for mechanical skin resurfacing according to embodiments of invention described herein. The system 1 includes a motorized device 3 that is generally shaped to be held in a hand of user. The apparatus 3 may be of varying shapes and dimensions, and one notable shape includes a substantially tubular or cylindrical body 5. The apparatus 3 generally includes one or more surfaces 7 for removably attaching a skin-contactable element 9 thereto. The term, "removably attaching", and variants thereof, relate to the ability to attach, remove, and reattach the element without significantly compromising the attachment strength. The skin-contactable element 9 (e.g., a sponge, a fibrous material or other material, or combinations thereof, including those described in this specification, below) includes a skin-contactable surface 11 for contacting the skin. The skin-contactable element 9 may be a part of a module 15 that includes the skin-contactable element 9 and an optional carrier 13. The optional carrier 13 (e.g., a firm plastic substrate) may be useful for removably attaching and detaching (e.g., via snap, threaded screw, friction fit or otherwise) the skin-contactable element 9 to the one or more surfaces 7 of the apparatus 3. A user grasping the body 5 may activate a motor (not shown in Figure 1) within the apparatus 3, such as by actuating a switch 17 on the body 5. The motor, thereby activated, provides mechanical energy that is transmitted to the attached skin-contactable surface 11 and to an expanse of skin 19 (shown in phantom in Figure 1) placed in contact therewith. The mechanical energy may be of various forms (e.g., vibration, rotation, reciprocation, and the like) that are transmitted via various means, e.g., an eccentric weight, a reciprocating shaft, and a rotating disc, among other means. The body 5 is generally shaped to facilitate easy grasping by the user so that the apparatus 3 is orient such that the attached skin-contactable surface 11 can contact the user's skin.

In order to permit mechanical energy from the apparatus 3 to be readily, predictably, and comfortably transferred through the skin-contactable element to the skin and still permit the pad to conform to a variety of skin surfaces, including those skin surfaces that are curved or angled, the inventors have recognized that one or more of certain properties of the skin-contactable element are highly desirable. As such, skin can surprisingly be abrasively treated using pressure that is largely governed by the apparatus 3, to provide benefits such as cell proliferation, microdermabrasion efficacy, cleansing, and the like without causing undo damage to the skin, or problems rinsing loose abrasive from the skin. Furthermore, using the inventive skin-contactable element, unwelcome microbial growth within the element is limited.

The inventors have surprisingly found that one or more of the above-mentioned desirable attributes may be achieved by using moderately abrasive skin-contactable elements that have an appropriate Abrasiveness, either "Durable Abrasiveness" or "Plain Abrasiveness" determined according to the "Abrasiveness Test" described in the "Test Methods" section, below. Furthermore, the above-mentioned benefits are further enhanced if the skin-contactable elements are selected based upon their Abrasiveness in combination with one or more properties relating to how the pads behave under compressive load, specifically Compressibility and Displacement. These properties relate to the ability of the skin-contactable element to transfer the mechanical energy from the apparatus 3 to the skin-contacting surface in a moist or wet environment to mechanically resurface the skin.

In one embodiment, the skin-contactable element has a Durable Abrasiveness has a Durable Abrasiveness from 2 to 14, preferably from about 2.5 to about 12, more preferably from about 3 to about 10, even more preferably from about 4 to about 9.

The inventors have also found that moderately abrasive pads may be selected based upon their "Plain Abrasiveness." In one embodiment, the skin-contactable element has a Plain Abrasiveness from 1 to about 5, preferably from 1 to about 2.

The inventors have also surprisingly found that moderately abrasive skin-contactable elements - particularly those meeting the fixed abrasiveness criteria as specified above, have enhanced performance when used in conjunction with a mechanical tool, when the skin-contactable element also has additional properties relating to their behavior under applied compressive load. In particular, the skin-contactable element provide some displacement under an applied load, but do not displace overly so.

Displacement of generally recoverable deformation due to an applied compressive force and Compressibility are additional properties useful to characterize the skin-contactable elements. These properties may be measured according to the "Compressibility and Displacement Test" described in the "Test Methods" section, below.

As such, in one embodiment, the skin-contactable element has a Displacement from 0.15 mm to about 2.0mm, preferably from about 0.25mm to about 1mm, more preferably from about 0.25 to about 0.8mm, and most preferably from about 0.25mm to about 0.5mm.

The inventors have also surprisingly found that moderately abrasive skin-contactable elements is compressible, but not overly so. As such, in one embodiment, the skin-contactable element has a Compressibility of less than about 20%. In other embodiments, the Compressibility may be less than about 19%, or more preferably less than about 15%. Most preferably, the Compressibility may range from about 3% to about 13%.

The inventors have also noted that it is desirable for the skin-contactable element to have a thickness that is from about 0.1mm to about 20mm, preferably from about 0.5mm to about 5mm, more preferably from about 1mm to about 5mm, and most preferably from about 1.5mm to about 4.5mm. Thickness may be determined as the "Initial Thickness" in the Compressibility and Displacement Test, below.

Aside from the inventors findings regarding desirable Abrasiveness properties and desirable behavior under applied compressive load, the inventors have also noted that it is desirable for the skin-contactable element to have surface that is rough, but not overly so.

In one embodiment, the article has a maximum surface roughness from about 200 microns to about 3000 microns, preferably from about 300 microns to about 2000 microns, more preferably from about 350 microns to about 1500 microns, and even more preferably from about 400 microns to about 1200 microns. The article may include a fibrous material, such as one having an abrasive system bound thereto.

In another embodiment, the article has an average surface roughness from about 25 microns to about 300 microns, preferably from about 30 microns to about 200 microns, more preferably from about 35 microns to about 150 microns, and even more preferably from about 50 microns to about 100 microns.

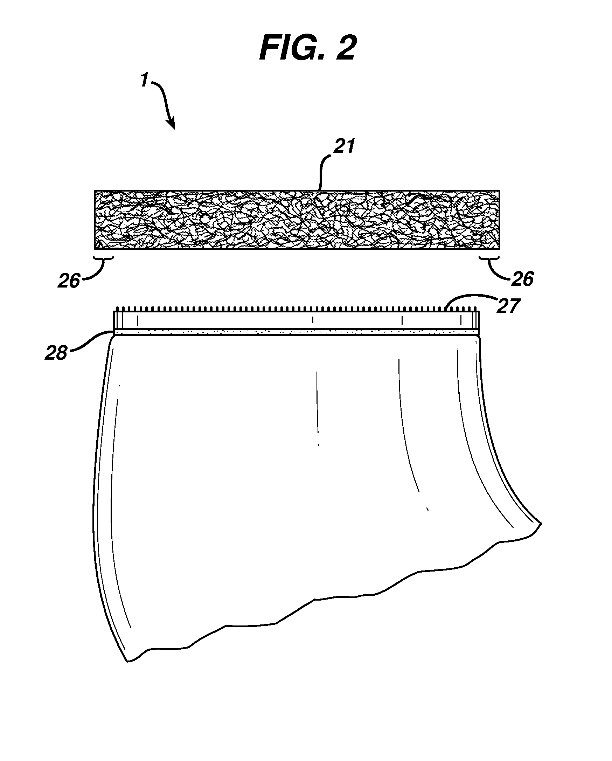

Figure 2 depicts an embodiment of system 1 in which a skin contactable element 29 comprises, consists essentially of, or consists of a fibrous material. Suitable fibrous materials include, without limitation, woven, nonwoven (oriented, e.g., via a carding process, or non-oriented), or knit fabrics. The fibers may be integrated into a nonwoven structure via, for example, needle punching, through-air bonding, hydro entangling, spun-bonding, chemical bonding (including adhesive bonding), or mechanical processing (such as embossing). The fibers may thereby be arranged into a freestanding fabric (e.g., a porous fabric). The nonwoven fabric may have an average pore diameter (as calculated via Cohen, "A Wet Pore-Size Model for Coverstock Fabrics," Book of Papers: The International Nonwovens Fabrics Industry, pp.317-330,1990) that is from about 150 microns to about 500 microns, such as from about 220 microns to about 400 microns. A representative, non-limiting list of useful fibers includes fibers derived from organic polymers such as, for example, polyester, polyolefin, polyamide and rayon fibers and bicomponent fibers; cellulose-based fibers such as wood pulp, rayon, and cotton; and combinations thereof.

The inventors have found, in one embodiment of the invention, in order to provide an appropriate degree of Abrasiveness and Compressibility, the fibers are bonded via mechanical means such as a needle-punching process, known to those skilled in the art, such as to a thickness of about 0.5mm to about 5mm, more preferably from about 1mm to about 5mm. The fibrous material may have a basis weight (mass per unit area) sufficient to maintain its mechanical integrity for one or more uses of the skin contactable element 29. The basis weight may be, for example, between about 10 grams per square meter (gsm) and about 450 gsm, such as between about 200 gsm and about 400 gsm, preferably between about 300 and about 400 gsm. The fibrous material desirably includes rayon to provide softness and a strong, resilient material such as an olefin or polyester. One particularly notable fibrous material is a needle-punched blend of staple-length 1.5 denier "TENCEL" rayon and staple-length 4-5 denier PET available from Precision Custom Coating of Totowa, NJ, with a basis weight of about 200 gsm and about 400 gsm.

Referring again to Figure 2, the fibrous material of the skin-contactable element 29 may be capable of removably attaching and detaching to a loop-engageable surface 27 on the apparatus 3. Once the loop-engageable surface 27 and the skin-contactable element 29 are engaged, the loop-engageable surface 27 is generally capable of firmly holding the skin-contactable element 29 in place throughout the time period during which the skin-contactable element 29 is brought in contact with the skin 19.

In one embodiment of the invention, the skin-contactable element 29 may have a peel strength required to separate the skin-contactable element from a loop-engageable surface (such as VELCRO USA, as hook no. 108 described below, as measured using an Instron) that is from about 100 grams per inch width to about 400 grams per inch width, such as from about 150 grams per inch width to about 250 grams per inch width. In one embodiment of the invention, the loop-engageable surface 27 is relatively smooth and non-abrasive, such that if the skin-contactable element 29 is misaligned (i.e., a portion of the loop-engageable surface 27 is exposed and thereby capable of contacting the skin 19), the loop-engageable surface 27 is not overly harsh to the skin 19. In one embodiment of the invention, the skin-contactable element 29 includes a buffer region 26 that is designed to "overhang" the loop-engageable surface 27 such that it is less likely, even with some misalignment of the skin-contactable element 29 and the loop-engageable surface 27, for portions of the loop-engageable surface 27 to contact the skin in use. The skin-contactable element 29 may have an area for contacting the skin that is greater than about 5 cm$^2$ In one preferred embodiment, the skin-contactable element 29 has a skin-contacting area for contacting the skin that is from about 5-50 cm$^2$ and more preferably about 11-50 cm$^2$

The loop-engageable surface 27 may be secured onto the apparatus 3 in a permanent, irreversible manner such as by a layer 28 of adhesive. Alternatively, the loop-engageable surface 27 may be detachably/ reattachably secured to the apparatus 3 as described in sections of this document below.

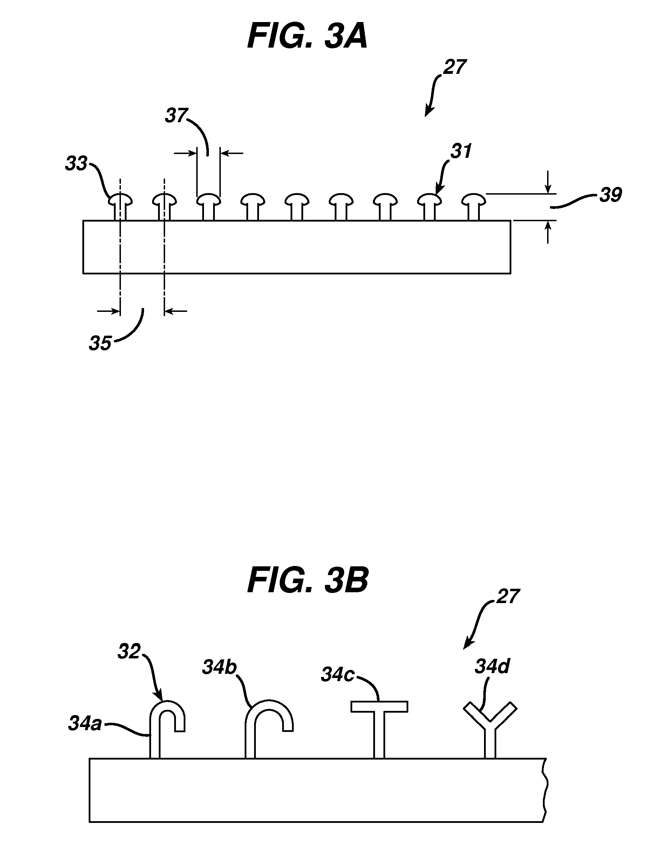

As shown in Figure 3, the loop-engageable surface 27 generally includes a plurality of protrusions 31. While various shapes of the protrusions 31 are contemplated, in order to promote both firmness of hold to the skin-contactable element 29 during use, as well as ease of release from the skin-contactable element 29 when a user intentionally attempts to pull on the skin-contactable element 29 to detach it from the loop-engageable surface 27. The protrusions 31 may be rounded, such as mushroom-shaped, as shown in Figure 3A or the protrusions may have other rounded shapes, such as shown in Figure 3B, for example, curled (protrusion 34a), arcuate (protrusion 34b), T-shaped (protrusion 34c), Y-shaped (protrusion 34d) or otherwise configured to provide a high surface area per protrusion that may contact the skin 19 that abuts the loop-engageable surface 27. In another embodiment, the protrusions 31 are bent, angular, forked, hook-shaped, or the like to provide a somewhat stronger hold to the skin-contactable element 29.

Furthermore, in another embodiment of the invention, in order to promote softness, a height 39 of the protrusions may relatively high, such that the skin 19 is less likely to feel discomfort in the situation where the loop-engageable surface 27 comes into contact with the skin 19. For example, the protrusions 31 may have height 39, e.g., an average height, that is greater than about 0.05mm, such as greater than about 0.10mm, such as from about 0.15mm to about 0.5mm.

In one embodiment of the invention, the protrusions 31 have a spacing 35, e.g., an average unit spacing (center-to-center distance between a protrusion and its nearest neighbor considered from a top view) that is less than about 5mm, more preferably less than about 2mm, most preferably less than about 1mm. In another embodiment of the invention, the protrusions 31 are present in a number density, e.g., an average number density, that is greater than about 0.25 protrusions per square millimeter (0.25/mm$^2$), more preferably greater than about 0.50/mm$^2$.

Referring again to Figure 3A, in another embodiment of the invention, also to promote softness and comfort upon inadvertent contact with the skin 19, the protrusions may have head regions 33 of the that have a diameter 37 that is relatively large. In one embodiment of the invention, the head regions 33 of the protrusions 31 have a diameter 37 that is greater than about 0.05mm, more preferably greater than about 0.2mm, most preferably from about 0.3mm to about 1mm.

In one embodiment of the invention, the protrusions 32 are configured such that each has a surface area capable of simultaneously contacting the skin that is at least about $0.002 \text{ mm}^2$, such as at least about $0.02 \text{ mm}^2$, such as from about $0.2 \text{ mm}^2$ to about $\text{mm}^2$.

In yet another embodiment of the invention, the protrusions 32 have both a number density greater than about $0.25/ \text{mm}^2$ and a height greater than about 0.05mm, more preferably a number density greater than about $0.5/\text{mm}^2$ and a height greater than about 0.1mm, even more preferably a number density greater than about $0.5/ \text{mm}^2$ and a height greater than about 0.15mm.

Although various loop-engageable surfaces 27 may be suitable, one suitable fastener is commercially available as VELCRO, from Velcro USA, of Manchester, New Hampshire and has a mushroom shape protrusions with an average height of about 0.17mm, an average unit spacing of about 0.4mm; an average head diameter of 0.69mm; and an a number density of about $5/\text{mm}^2$. Note that for protrusions that have a head that is non-circular, as viewed from the top, an equivalent head diameter ("$D_{eq}$") may be calculated from the measured head area ("A") as follows:

$$D_{eq} = (4A / \pi)^{\frac{1}{2}}.$$

Another suitable loop-engageable surface 27 has "Y"-shaped protrusions with an average height of about 0.37 mm (overall height) an average unit spacing of about 0.84mm; an average head diameter of 0.37 mm; and an a number density of about 0.75/mm (commercially available from VELCRO USA, as hook no. 108).

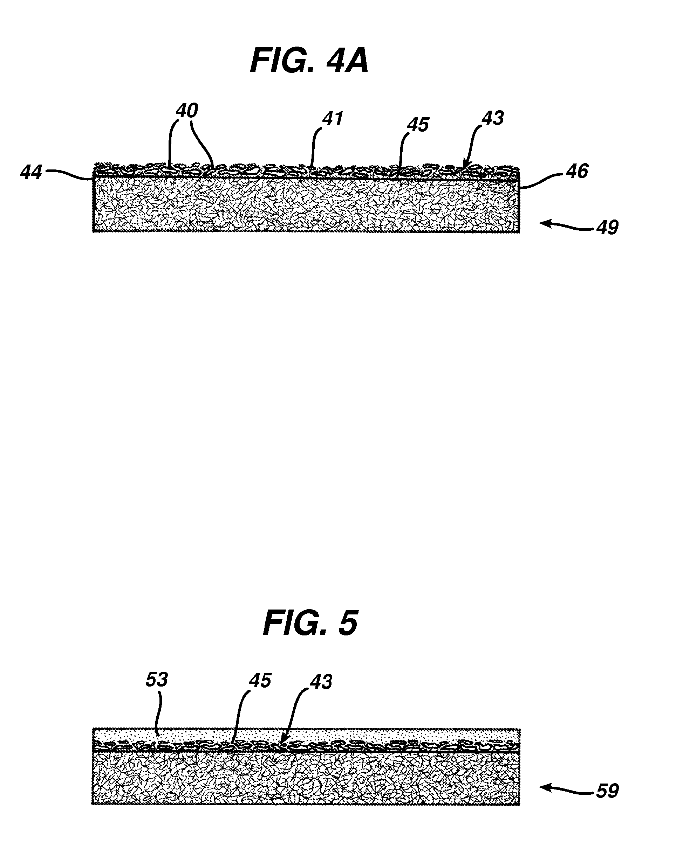

Figure 4A depicts a cross sectional view of another embodiment of a skin-contactable element 49. The skin-contactable element 49 is similar to the skin contactable element 29 depicted in Figure 2; however, the skin-contactable element 49 includes an abrasive system 43 bound to a network of fibers 45.

The term an abrasive system "bound to fibers" refers to abrasive units, particles, aggregates, and the like that are firmly attached to the fibers and do not readily separate in use therefrom. Such abrasive may be bound by various means; one notable means is by chemical bonding (including, without limitation, adhesive bonding).

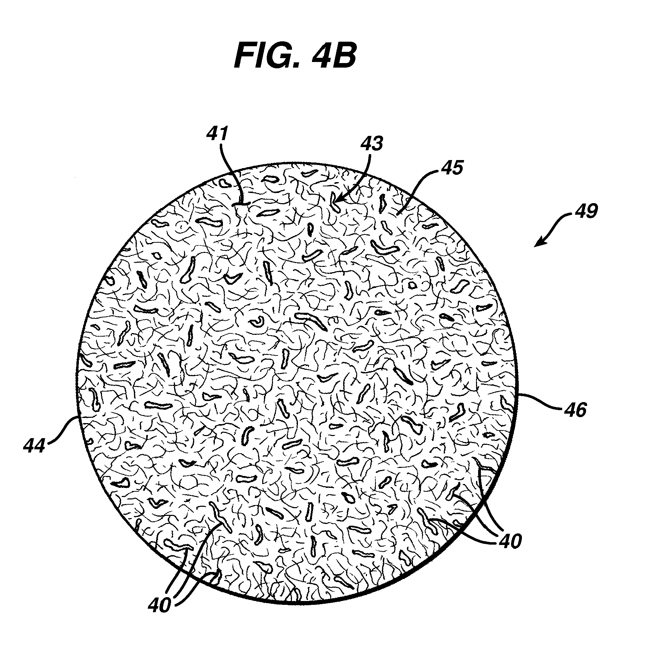

The inventors have noted that, as depicted in Figure 4A (cross-sectional view) and Figure 4B (top view), according to one embodiment of the invention, the abrasive system 43 may include a plurality of discrete abrasive units 40, such as may be distributed among and/or across the fibers 45. In this embodiment of the invention, a skin contactable surface 41 includes both fiber and abrasive system. This configuration may provide better microdermabrasion efficacy than the configuration in which the abrasive system 43 is a continuous layer formed entirely across the fibers, extending continuously from one end 44 of the skin contactable element to a opposite end 46.

The discrete abrasive units 40 of the abrasive system 43 may be of varying shapes, e.g., substantially spherical, dendritic, and the like. The abrasive units 40 may have a maximum end-to-end dimension (i.e., the length of the longest line that can be drawn within one discrete abrasive unit 40) that is, for example, from about 0.2mm to about 1cm.

The abrasive system 43 may include or consist essentially of a water insoluble abrasive material such as an abrasive having a Mohs hardness of less than about 4. In one embodiment of the invention, the abrasive system includes a resin or polymer. For example, the polymer may be a homopolymer, copolymer, or terpolymer, and may be a blend of two or more different polymers. The polymers may be random, block, star, or other known architecture. The polymer may be made by known means, such as emulsion polymerization, dispersion, suspension, or solution polymerization. In a preferred embodiment the polymer is formed by emulsion polymerization. The polymers may be non-functional, or may contain functionality designed to optimize the properties of the coating in the specific application. One of skill in the art will be able to adjust monomer content and architecture to improve end-use performance of the polymer composition. The polymer could be a synthetic polymer, or could be a natural polymer such as, for example, a polysaccharide, starch, modified starch, or guar gum. Preferred polymers include homopolymers and copolymers having one or more of the following monomers: (meth)acrylates, maleates, (meth)acrylamides, vinyl esters, itaconates, styrenics, unsaturated hydrocarbons and acrylonitrile, nitrogen functional monomers, vinyl esters, alcohol functional monomers. Particularly preferred monomers include, but are not limited to, vinyl acetate; methyl (meth)acrylate, ethyl (meth)acrylate, butyl (meth) acrylate, ethylene, vinyl chloride, and styrene.

If included in the skin contactable element,, the polymer is selected so as to provide enough hardness so as to be abrasive to skin, but not so hard as to cause scratching or discomfort. In one embodiment of the invention, the polymer has a glass transition temperature, $T_g$ greater than about -20 degrees Celsius (° C.), such as from about 0° C. to about 105° C. In one notable embodiment, the polymer has a $T_g$ from about 0° C and about 50° C.

$T_g$ can be determined by differential scanning calorimetry (DSC) conducted at a heating rate of 20.0 °C./minute with 5 mg or smaller samples. The $T_g$ is calculated as the midpoint between the onset and endset of heat flow change corresponding to the glass transition on the DSC heat capacity heating curve. The use of DSC to determine $T_g$ is well known in the art, and is described by B. Cassel and P. DiVito in "Use of DSC To Obtain Accurate Thermodynamic and Kinetic Data", American Laboratory, January 1994, pp 14-19, and by B. Wunderlich in Thermal Analysis. Academic Press, Inc., 1990.

The polymer may be a thermosetting polymer, (e.g., a polymer having crosslinks that are generally not reversible with changes in temperature). One notable polymer has an acrylic base/ vinyl acrylic base that is partially cross-linked during cure with a Tg of about 30° C., e.g., VINAMUL ABX 30, resin commercially available as from Celanese Corporation of Dallas, TX.

Applicants have noted that, in one embodiment of the invention, in order to provide a proper balance of skin treatment efficacy without causing a perception of harshness to the skin, the abrasive system preferably includes a polymer having a $T_g$ from about 0° C. to about 50° C. Furthermore, Applicants have also noted that the abrasive system 43 that includes the polymer having a $T_g$ from about 0° C. to about 50° C. is desirably present on the fibers such that the weight ratio of abrasive system to fiber is from about 5% to about 30 %, more preferably from about 8% to about 23%, even more preferably from about 8% to about 18%, and most preferably from about 8% to about 12%.

While above, the abrasive system 43 is described as including a polymer, a polymer need not be present in the abrasive system 43. The abrasive system 43 may derive its abrasiveness from other means. For example, the abrasive system 43 may include an inorganic particle (e.g., aluminum oxide, pumice, and the like) that is bound to the fibers 45, such as by chemical bonding (e.g., via an organosilane, or via a polymer that is itself abrasive) or thermal bonding. In one embodiment, in order to reduce irritation to the skin, the inorganic particle has a Mohs harness of 3 or less, such as talc, gypsum, mica, or calcite.

The abrasive system 43 may further include one or more additional functional components compounded with the abrasive. Useful additional functional components include, but are not limited to plasticizers; cross-linkers; starch; polyvinyl alcohol; formaldehyde thermosetting agents such as melamine, urea, phenol; fillers; humectants; surfactants; salts; fragrances; and pigments or reflective agents. The additional functional components may be present in the abrasive system at from 0 to 20 percent by weight, and preferably from 5 to 15 percent by weight, calculated as a percent of the polymer solids.

The skin contactable element 49 may be formed by depositing the abrasive system 43 onto the fibers 45 by various means known to the art of industrial polymer coating, such as slot coating, foam coating, saturation, printing, or spraying. Spraying is particularly notable to facilitate the formation of discrete abrasive units on top of the fibers so that waste is reduced and efficacy is optimized. If the abrasive system 43 is applied by spraying, a sprayable composition that includes the abrasive system (e.g., polymer plus other functional ingredients as well as water or another suitable carrier) may be sprayed onto the fibers followed by drying the resulting fiber/abrasive composite in a conventional oven. Although, the foregoing relates to a skin-contactable element that incorporates an abrasive system bound to the fibers, in one embodiment of the invention, the fibers themselves may be abrasive, without the need of including an additional abrasive system bound to the fibers. For example, in one particular embodiment, the skin-contactable element includes staple fibers that are integrated into a nonwoven structure via needle punching, through air bonding, or thermal bonding. The fibers may be high denier fibers formed from polyester; polyolefins; rayon fibers; bicomponent fibers; cellulose-based fibers such as wood pulp, rayon, and cotton; or combinations thereof.

One particular non-limiting example of skin-contactable element in which the fibers themselves provide the Abrasiveness (e.g., no abrasive system bound to the fiber is present) is one which includes (1) polyester fibers having a denier from about 5 to about 10, such as about 9, and a length from about 1 inch to about 2 inches; or (2) bicomponent fibers having a polyester or polypropylene core and a polyethylene core; with a denier from about 2 to about 6; or combinations thereof.

In another embodiment of the invention, the fibrous structure includes a layer of foam or other resilient material. For example, a laminate consisting of the nonwoven material described above with abrasives and formulations (call it material A), plus an added layer of foam material (call it B) for added loft and softness. The laminates could be arranged in several arrangements; A:B or A:B:A, or the B material could be under the loop-engageable fastener for some added compressibility for the system. One surface of B could be coated to be water/formulation impervious to prevent sucking up formulation.

In another embodiment of the invention, the skin-contactable element includes an apertured plastic film for providing abrasion to the skin. In this embodiment of the invention, the skin-contactable element may include or be free of fibers. For example, in one particular embodiment, the skin-contactable element includes a film such as one formed from an olefinic material such as polyethylene or polypropylene. In order to provide sufficient abrasion to the skin, high-density polyethylene and polypropylene are particularly preferred. Furthermore, the apertured plastic film may have a thickness prior to aperturing that is greater than about 1 mil, such as from about 1.5 mils to about 3 mils. Furthermore, to enhance abrasion, the film has apertures formed through the film and including protrusions that extend beyond a plane of the film thickness. The protrusions are designed to contact the skin of the user and to provide abrasion thereto. In order to provide a sufficient number of contact points for the skin, the apertured plastic film may have a plurality of apertures, such as may be generated by having an open area from about 20% to about 35%. The apertured plastic film may be formed by any of various methods known to the art (e.g., direct extrusion, vacuum, among others). A composite structure incorporating an abrasive surface may be formed by attaching a barrier film to a side of the apertured film that is oriented away from the skin of the user. One or more benefit agents may be contained within composite structure such that when the apertured contact the skin, the benefit agents are released from the composite structure and are available to contact or be absorbed by the skin.

Figure 5 depicts a cross sectional view of another embodiment of a skin-contactable element 59. The skin-contactable element 59 is similar to the skin contactable element 49 depicted in Figure 4, however, the skin-contactable element 49 includes a coating 53 formed about or across the fibers 45, and, in one embodiment, as shown in Figure 4, formed atop the fibers 45 and atop the abrasive system 43 as well. The coating 53 may be least partially water-soluble such that in use, one or more ingredients within the coating 53 dissolve in use and are transferred to the skin.19. In one embodiment of the invention, the coating 53 is substantially free of abrasive, such as abrasive particles that could be transferred to and embed in the skin. In one embodiment of the invention, the coating 53 is substantially free of water (i.e., includes less than about 2%, such as less than about 0.5% of water).

The coating may be formulated for one or more of various functions. For example, the coating may provide lubrication, emolliency or and/or moisturization; mild foaming; a vehicle to deliver various benefit agents (e.g., benefit agents, drugs, and the like); or combinations thereof. Figure 5 depicts the embodiment wherein the coating 53 is a continuous coating that completely covers the fibers 45 and the abrasive system 43. In this embodiment of the invention, skin contactable surface 41 initially includes only coating 53. However, as the coating 53 dissolves, which can be quite rapid when placed in contact with moist skin, it permits the fibers 45 and abrasive system 43 to contact the skin.

The coating 53 need not be continuous and need not entirely cover either the fibers 45 or the abrasive system 43. As viewed from the top (not shown), the coating 53 may cover a significant portion of the entire top of the skin-contactable element 59, such as greater than about 20%, but less than 100%. In this embodiment of the invention, skin contactable surface 41 includes fibers 45, abrasive system 43, and coating 53.

The coating 53 may include various ingredients for conditioning and/or cleansing and/or providing foam. For example, the coating may include so-called "foaming" or "lathering" surfactants. As used herein, "lathering surfactant" means a surfactant, which when combined with water and mechanically agitated, generates a foam or lather. Such surfactants are preferred since increased lather is important to consumers as an indication of cleansing effectiveness. A wide variety of lathering surfactants are useful herein and include those selected from the group consisting of anionic lathering surfactants, nonionic lathering surfactants, cationic lathering surfactants, amphoteric lathering surfactants, and mixtures thereof.

For a detailed description of suitable formulations that may be used for the coating 53, the reader is referred to co-pending patent application, serial number 11/023655, filed December 28, 2004, entitled "SKIN TREATMENT ARTICLES AND METHODS," and, in particular, those sections entitled, "CLEANSING FORMULATIONS," "ANIONIC LATHERING SURFACTANTS," "NON-IONIC LATHERING SURFACTANTS," "CATIONIC LATHERING SURFACTANTS," "AMPHOTERIC LATHERING SURFACTANTS," "CONDITIONING FORMULATIONS," "HYDROPHOBIC CONDITIONING AGENTS," "HYDROPHILIC CONDITIONING AGENTS," "STRUCTURED CONDITIONING AGENTS," and "OTHER FORMULATIONS" herein incorporated by reference.

Furthermore, the coating 53 may include one or more benefit agents such as anti-acne agents, anti-wrinkle agents, anti-microbial agents, anti-fungal agents, antiinflammatory agents, topical anesthetic agents, artificial tanning agents, accelerator agents, anti-viral agents, enzyme agents, sunscreen agents, anti-oxidant agents, skin exfoliating agents, depilatory agents, and the like. Other suitable benefit agents are described in co-pending published patent application US2005-0148907, filed December 24,2003, entitled "TREATMENT OF SKIN USING A BENEFIT AGENT AND AN APPARATUS," and co-pending patent application serial number 11/023655, filed December 28, 2004, entitled "SKIN TREATMENT ARTICLES AND METHODS, both cited previously.

To enhance shelf-stability and flexibility of choices for packaging, the coating 53 may be substantially free of water (in this case, the pad could be wet with water before use). Alternatively, the coating and skin-contactable element 59 include substantial water or moisture and may be sealed in suitable packaging to prevent water loss to the external environment before use.

The coating 53 may be applied to the fibers such that the weight ratio of coating to fiber is from about 25.0% to about 100.0 %, more preferably from about 25% to about 50%. The coating may be applied to the fibers or the fiber/abrasive composite by slot coating, foam coating, saturation, nip roll, and the like.

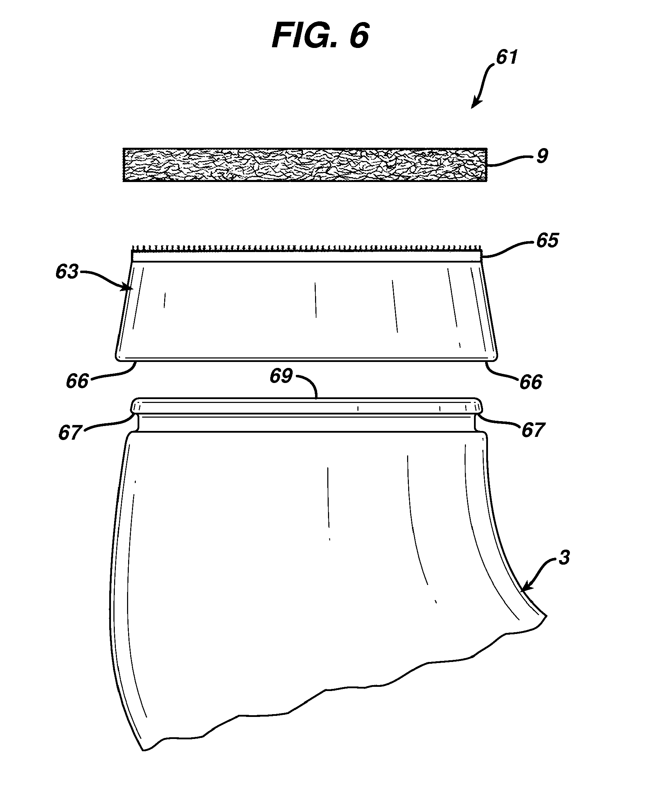

Figure 6 depicts another suitable embodiment of a system 61 for treating the skin. The system 61 includes motorized apparatus 3, as described with reference to Figure 1. System 61 further includes skin-contactable element 9, such as any of the skin-contactable elements discussed thus far. The skin-contactable element 9 is reversibly couupleable to the apparatus 3 using an adaptor 63. The adaptor 63 includes a first attachment 65 for reversibly attaching the skin-contactable element 9 to the adaptor 63.

The first attachment 65 is generally capable of firmly holding the skin-contactable element 9 to the adaptor 63 while the system is in use. Furthermore, the first attachment 65 is preferably able to maintain its hold upon exposure to moisture and water. For example, the first attachment 65 may be "water-resistant."By water-resistant, it is meant that if the first attachment is immersed in water for 30 minutes and then dried completely, no substantial loss in attachment strength is observed. In one notable embodiment of the invention, in order to reduce susceptibility to water, the holding power of the first attachment 65 is includes a means other than adhesives that may be softened or dissolved by water. The first attachment 65 may include, for example, a loop-engageable surface, such as the loop-engageable surface 27 described with reference to Figure 2, as a permanently bonded integral part of the adaptor 63. The loop-engageable surface may be permanently bonded to the remainder of the adaptor 63 by various means such as a durable water-resistant adhesive, such as may be coated on faces of double-sided tape.

The adaptor 63 includes a second attachment 66 for reversibly attaching the adaptor 63 to a surface of the apparatus 3. The adaptor 63 may be designed to reversibly attach to one or more of various surfaces of the apparatus 3. The surfaces suitable for attachment, include, but are not limited to a surface such as surface 69 that may be substantially parallel to the skin during use, or a surface such as surface 67, that forms a rim suitable for snap-fitting onto the apparatus 3, or a surface (not shown) that is internal to the apparatus 3, such as one that may be reversibly attached to a portion of the adaptor 63 (e.g., a protruding rod) that protrudes into a recess in the apparatus 3.

The first attachment 65 and the second attachment 66 generally have sufficient strength to substantially maintain a position of the skin-contactable element relative to the surface of the apparatus when the skin-contactable element is urged against the skin, and preferably sufficient strength to maintain position when the motor is empowered and the skin-contactable element is glided across the skin.

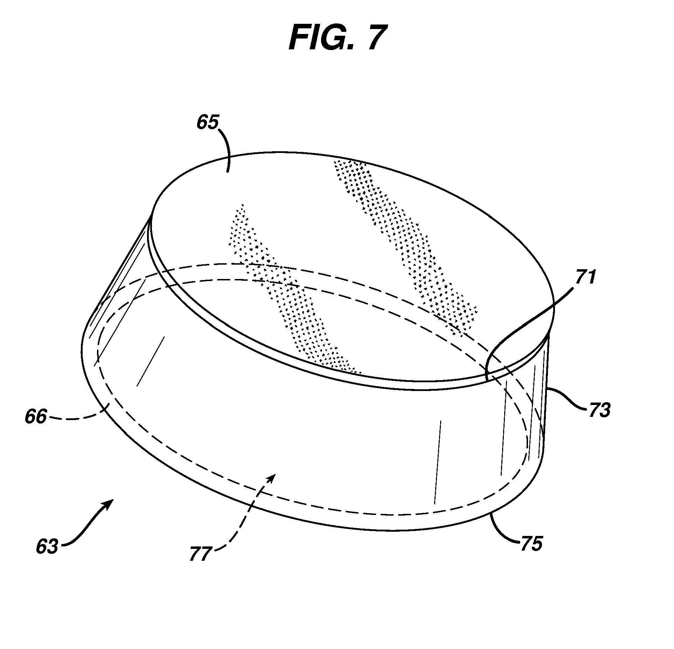

Figure 7 depicts one notable embodiment of the invention in which the adaptor 63 includes a substantially flat surface 71 that is generally positioned parallel to the skin in use. Permanently attached to the surface 71 is first attachment 65 for reversibly attaching the adaptor 63 to skin-contactable element 9. A frusto-conical flexible wall 73 extends from surface 71 and terminates in a circular rim 75. The rim 75, together with the wall 73, and the surface 71 define a hollow recess 77. The flexible wall 73 or the rim 75 may include a protruding feature (e.g., knobs, notches, ledges, and the like) that comprise second attachment 66 and aid in reversibly securing the adaptor 63 to the apparatus 3. This embodiment of adaptor 63 may be fabricated from a thermoplastic material, such as a hard plastic (e.g., polyethylene and the like or a softer plastic such as PETG or polystyrene, using various suitable processes for shaping of plastics, such as, for example thermoforming, injection molding, and the like.

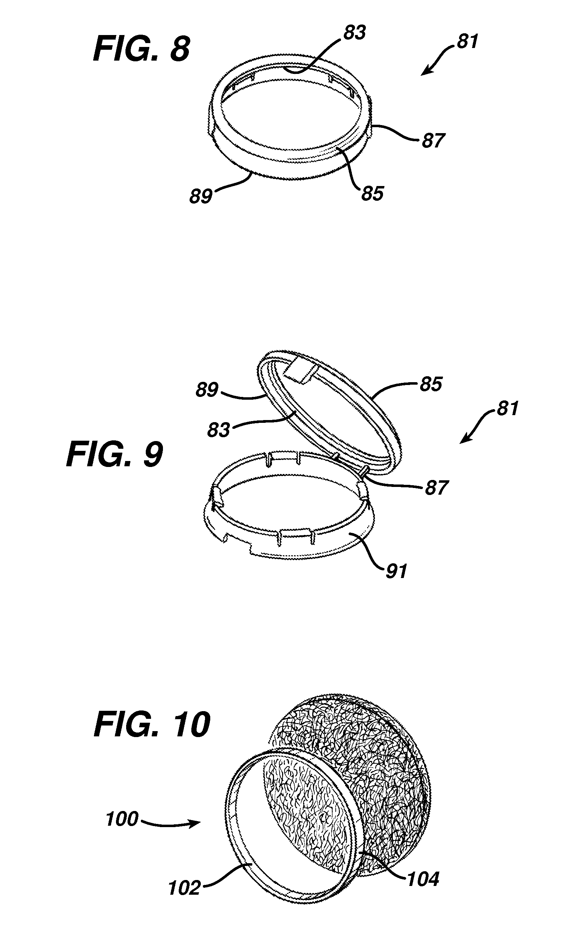

Figures 8 and 9 depict another embodiment of a suitable adaptor. Adaptor 81 is a clamp for holding the skin contactable element against a surface of the apparatus 3. The adaptor 81 has a first attachment 83 for reversibly attaching the adaptor 81 to the skin-contactable element. First attachment 83 is the underside of a circular rim 85. Adaptor 81 may have a hinge 87 to allow a user to rotate a hinged portion 89, thereby "opening" the adaptor 81. The user then places the skin-contactable element against a surface (cf. surface 69 of Figure 6) of the apparatus 3. The adaptor 81 has a second attachment 91 for reversibly attaching the adaptor 81 to the apparatus 3. The second attachment may be a threaded surface, a surface that is snappable or friction-fittable onto a corresponding surface (cf. surface 67 of Figure 6) of the apparatus 3.

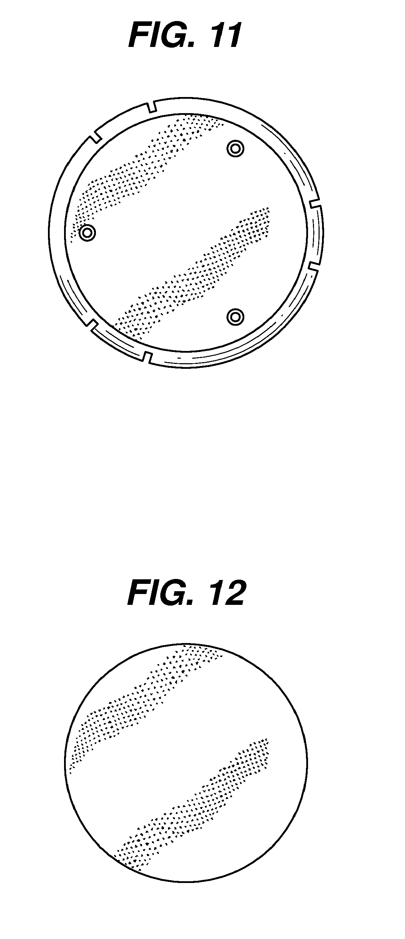

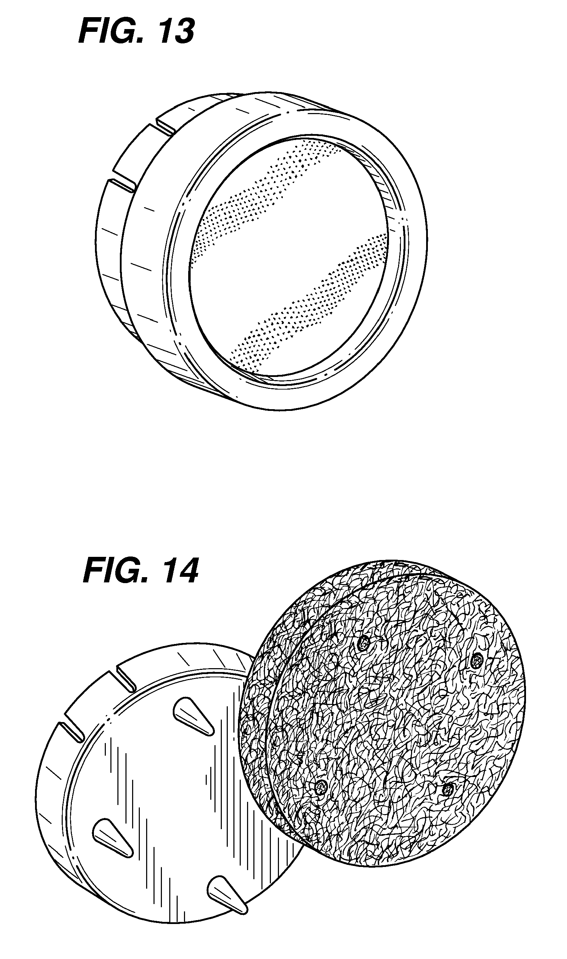

Figure 10 depicts another embodiment of an adaptor 100 formed of a resilient ring that holds the skin contactable element against a surface of the apparatus. The adaptor 100 includes a first attachment 102 to attach the adaptor 100 to the skin contactable element and a second attachment 104 to attach the adaptor 100 to the apparatus. Figures 11-14 illustrate additional embodiments of the adaptor.

Applicants have noted that adaptor 63 is particularly useful in reducing the manufacturing cost for treatment of the skin using apparatus 3. For example, by including adaptor 63 as apart of the system 1, the first attachment 65 (e.g., loop-engageable) need not be permanently affixed to the apparatus 3. As such, if the first attachment 65 is subject to wear in use, the entire apparatus 3 (the component of the system 1 that is generally most costly to produce) need not be disposed of. Instead, the user need only replace the adaptor 65 (less expensive that the apparatus 3). Furthermore, embodiments of the adaptor also provide resistance of attachments 65, 66 to damage from water.

## METHODS OF USE

System 1 of the present invention may be used to treat the skin, such as abrasive treatment, cleansing, or other skin treatments (e.g., acne, anti-aging, firmness, tone and texture, hair removal, body shaping/cellulite removal, and the like).

In one embodiment of the invention, the skin-contactable element is temporarily attached to the hand-held motorized apparatus (see, for example, Figure 2). In an alternative embodiment, the adaptor is removably/replacably attached to the apparatus and the skin-contactable element is reversibly attached to the adaptor (see, for example, Figure 7).

The motor is then empowered, and the skin-contactable element is moved across the face or other expanse of skin to be treated. For example, a skin-contacting surface 21 (e.g., a substantially planar skin-contacting surface) is placed into contact with the skin to be treated. The skin-contactable element provides, for example, increased cell proliferation by abrasively treating the skin. The skin-contactable element may have incorporated therewith a formulation to provide emoliency, foam, or delivery of benefit agents to the skin. When the user is finished, the skin-contactable element may be removed and later replaced with a fresh one to provide a hygienic surface.

The system may be used with an additional composition (e.g., a cream or paste) to provide lubrication, deliver actives, or provide an overall aesthetic experience. The composition may be free of abrasives (pumice, oxides. etc.) that would otherwise potentially embed in the skin. Alternatively, the composition may include abrasives, however, in this embodiment, the user would preferably rinse the abrasive composition from the skin after the treatment is complete. The composition may be placed by the user (e.g., by dipping the skin-contactable element into the cream) on the skin-contactable element prior to empowering the apparatus.

The inventors have discovered that by employing the skin-contactable element as well as related methods, and systems of the present invention, mechanical energy can be readily, predictably, and comfortably transferred through the skin-contactable element to the skin and still permit the pad to conform to a variety of skin surfaces, including those that are curved or angled. As such the skin can surprisingly be abrasively treated using controllable pressure to provide benefits such as cell proliferation, microdermabrasion efficacy, cleansing, and the like without causing undo damage to the skin or resulting in problems with rinsing loose abrasive from he skin. Furthermore, these embodiments can provide a disposable, hygienic, skin-contactable element that is economical to manufacture. Furthermore, the skin-contactable element may serve additional functions beyond providing abrasion, such as delivery of benefit agents, lubrication, and lathering.

The invention also permits abrasive treatment of the skin without the potential mess and inconvenience of a using an apparatus with a cream having dispersed abrasives particles, which may adhere to and embed in the skin.

## TEST METHODS

### ABRASIVENESS TEST

"Durable Abrasiveness" is determined using the test method described below. The "Plain Abrasiveness" of a material is determined similarly to the Durable Abrasiveness, but the initial washing step is eliminated.

Five (5) samples of each of the skin-contactable elements to be tested are cut to a circular shape having a diameter of about 41 mm. Samples are individually rinsed with water in order to remove any materials such as foaming agents, oils, and emulsifiers that are readily separated from the article via contact with water. The cut samples are immersed in a bath of containing a mass of deionized water (temperature of about 35°C) that has sufficient mass of water to be at least about 20 times the mass of the article. The article is allowed to remain in the bath for two minutes and is removed, allowed to drip for 10 seconds and then placed in another similar (fresh) water bath for two minutes, and again allowed to drip for 5 minutes. The sample is removed and allowed to dry at ambient temperature (at about 50-60% relative humidity) for a period from about 16 hours to about 72 hours, Again, this washing step is eliminated when measuring the Plain Abrasiveness.

After the sample is rinsed and dried as above, it is tested for abrasion using an abrasion testing device according to a modified version of ASTM test method D 3886-99, A suitable device is the CSI Universal Wear Tester, Model CS-226-605, available from Custom Scientific Instruments of Whippany, NJ. A sample of co-extruded spunbond/pigmented polyethylene film laminate (Clopay M18-1057, a 26 gsm laminate having (1) a 15 gsm (nominal) spunbonded polypropylene nonwoven web layer that is coextruded with (2) a 20 gsm (nominal) polyethylene film having a thickness of about 0.7 mils (0.007 inches), in which the polyethylene film surface of the laminate is corona treated, and the laminate has a target bond strength of 150 grams per inch, commercially available from Clopay Plastic Products of Mason, OH) is placed over the stage with the film oriented up, and the laminate is secured firmly against the stage with an o-ring, as supplied with the wear tester. The sample to be tested is secured on the arm above the stage such that it aligns directly on top of the stage. The sample is secured (preferably by tough double-sided tape - e.g., PERMACEL tape available from Permacel Company of East Brunswick, NJ in a manner such that the sample does not move when the tester is in operation. A 10 lb weight is loaded on the stage and the tester motor is powered. The stage simultaneously rotates and translates at a rate of about 130 cycles per minute. The number of cycles to failure is recorded as the first cycle in which the film is tom (for a pigmented, e.g., blue, film, the white spunbond readily shows through, marking the endpoint of the test. The process is repeated for the remaining samples. The average number of cycles to failure is recorded and a value for "Durable Abrasiveness" (for the washed samples) or for "Plan Abrasiveness" (for the unwashed samples) is calculated as 2000 divided by the average cycles to failure.

A standard sample, SCOTCH-BRITE Pad ("Heavy Duty Commercial Scoring Pad," # 86) is desirably run as a standard with each data set. The SCOTCH-BRITE Pad, # 86 should yield a Durable Abrasiveness value of approximately 33 +/- 4. If the operator determines a Durable Abrasiveness that falls outside this range, this signifies slight operator error, and the operator should adjust any subsequent determinations for Durable Abrasiveness by a factor that corrects for this operator error. That factor is (V/33), where V is the value determined by the operator for SCOTCH-BRITE Pad, # 86. If SCOTCH-BRITE Pad, # 86 is not available, then, as a substitute, SCOTCH-BRITE Pad ("Genera! Purpose Commercial Scoring Pad," #96") can be run as a standard, with the expected value of Durable Abrasiveness as 14 +/- 2 and a correction factor of(V/14) if this alternative standard does not fall within the prescribed range.

The Abrasiveness value for the five samples is averaged and reported as the Durable Abrasiveness or Plain Abrasiveness value for the particular skin-contactable element.

COMPRESSIBILTY AMD DISPLACEMENT <u>TEST</u>

"Displacement" is determined using the following test method: for each article to be tested, five samples are cut to a size of about 41 mm diameter. One at a time, a sample is placed on a thickness gauge such as the Ames Logic Plus (model LG3601-1-04) available from BC Ames of Waltham, MA, and the sample is centered under the 55mm foot. A 0.5 oz weight is placed on the shaft and the foot is gently lowered onto the sample. The "Initial Thickness" reading is taken after the gauge is allowed to stabilize for 10 seconds. Next, the foot is lifted, the 0.5 oz. Weight is replaced with an 8 oz weight. After the gauge is allowed to stabilize for 10 seconds, the "Thickness Under Load" is recorded. The process is repeated for 10 samples. For each sample the difference between Initial Thickness and Thickness Under Load is calculated and recorded. The result for the 1 0 samples is averaged and recorded as the Displacement for the particular skin-contactable element.

"Compressibility" is calculated as the Displacement of a sample divided by its Initial Thickness and expressed as a percent. The result for the 10 samples is averaged and recorded as the Compressibility for the particular skin-contactable element.

SURFACE ROUGHNESS TEST

Surface Roughness is readily determined using an optical instrument designed to measure surface features such as the Optical 30 Skin Measurement Device Primos Compact, commercially available from GF Messtechnik GmbH of Teltow, Germany. In order to determine both maximum and average surface roughness, one may utilize software commercially available with the device. A sample to be measured is placed on a flat surface such as a bench top (taped down if necessary), brought into focus, and a surface area of about 25mm to 30mm is scanned according to "star roughness," (radial) profile. An 8-rank polynomial is fit to the surface in order to determine the roughness parameters. Maximum surface roughness and average surface roughness are determined using the software interface.

EXAMPLES

The following examples relate to skin-contactable elements of the present invention. Other embodiments of the invention can be prepared in an analogous manner by a person of ordinary skin in the art.

Examples 1-3

A freestanding fibrous, non-woven material (a needlepunched blend of 55% lyocell and 45% polyester, having a basis weight of about 200 gsm and a thickness of about 2.5mm, available from Precision Custom Coating of Totowa, NJ, USA) was sprayed with an abrasive composition to form a skin-contactable element. The abrasive composition contained an abrasive system which was a blend of about 95.7% by weight of ABX 30 RESIN, (approximately 50% by weight of which is polymer), available from Celanese Corporation of Dallas, TX., about 4% mica (Prestige Sparkling Silver with a particle size from 20-150 microns, available from Ekhart America L.P of Painesville, OH) and about 0.3% of polyacrylic acid thickener, ALCOGUM 296W., available from Alco chemical. Sufficient water was added to permit the composition to be sprayed onto the non-woven to provide a concentration by weight of abrasive system to non-woven described in the Table below. The non-woven with the composition applied thereto was then dried in a conventional oven. The abrasive system was present to a large degree on the surface of the non-woven fibers.

| Example | wt-% abrasive system |
|---------|---------------------|
| 1 | 8 |
| 2 | 12 |
| 3 | 24 |

The non-woven/abrasive composite was then cut into circular pads having a diameter of 41 mm. A conditioning composition was then coated across the entire top surface of the composite non-woven/abrasive system. The conditioning composition included the following ingredients:

| Trade Name | Chemical Name | %(w/w) |
|---|---|---|
| Texapon NC70 | Sodium Laureth Sulfate | 8.7000 |
| Tegobetaine F-50 | Cocamidopropyl Betaine | 3.4800 |
| Plantaren 2000 N | Decyl Glucoside | 2.9000 |
| Monateric 949J | Disodium Lauroamphodiacetate | 4.0600 |
| Atlas G-4280 | PEG-80 Sorbitan Laurate | 11.6000 |
| Gluquat 125 | Lauryl Methyl Gluceth-10 Hydroxypropyldimonium Chloride | 0.5800 |
| Phenoxetol | Phenoxyethanol | 0,5220 |
| Nipa Butyl | Butyl Paraben | 0,0435 |
| Methyl Paraben | Methyl Paraben | 0.0899 |
| Propyl Paraben | Propyl Paraben | 0.0580 |
| Fragrance | Fragrance | 0.3480 |
| Citric Acid anhydrous | Citric Acid | 0.1160 |
| Carbowax PEG 400 | Citric Acid | 6.3626 |
| Emery 917 | Glycerin | 19.1400 |
| Frescolate ML Crystal | Menthyl Lactate | 2.0000 |
| Cutina WW9 | Cocoglycerides<br>Glyceryl stearate<br>Glyceryl laurate<br>Stearyl alcohol<br>Myristic acid | 40.0000 |

The conditioning composition was coated in the laboratory using a tongue depressor, on the non-woven/abrasive composite such that each 4" diameter pad had about 0.75 grams of conditioning composition coated thereon to form a skin-contactable element.

An adaptor was made by thermoformed PETG plastic, similar to the embodiment of the invention depicted in Figure 7. A loop engageable attachment was permanently bonded on the top flat surface of the adaptor. The skin-contactable element was mounted on an adaptor by placing the skin-contactable element on the loop-engageable surface and pressing firmly.

Example 4

A skin-contactable element was made in a manner identical to Example 2, except that a cleansing composition (detailed below) was applied to the non-woven/abrasive composite rather than the conditioning composition detailed above.

| Trade Name | chemical Name | % (w/w) |
|---|---|---|
| Texapon NC70 | Sodium Laureth Sulfate | 15.0000 |
| Tegobetaine F-50 | Cocamidopropyl Betaine | 6.0000 |
| Plantaren 2000 N | Decyl Glucoside | 5.0000 |
| Monateric 949J | Disodium Lauroamphodia | 70000 |
| Atlas G-4280 | PEG-80 Sorbitan Laureate | 20.0000 |
| Glucquat 125 | Lauryl Methyl Gluceth-10 Hydroxypropyldimonium Chloride | 1.0000 |
| Phenoxetol | Phenoxyethanol | 0.9000 |

(continued)

| Trade Name | chemical Name | % (w/w) |
|---|---|---|
| Nipa Butyl | Butyl Paraben | 0.0750 |
| Methyl Paraben | Methyl Paraben | 0.1550 |
| Propyl Paraben | Propyl Paraben | 0.1000 |
| Fragrance | Fragrance | 0.6000 |
| Citric Acid anhydrous | Citric Acid | 0.2000 |
| Carbowax PEG 400 | Polyethylene glycol | 10.9700 |
| Emery 917 | Glycerin | 33.0000 |

The PEG-80 Sorbitan Laurate and Disodium Lauroamphodiacetate were added together in a beaker and mixed until homogenous. The butylparaben, methylparaben, and propylparaben were added thereto and slowly mixed until the parabens dissolved. The PEG-8 and glucquat were then added to the beaker and mixed. The Cocamidopropyl Betaine, Sodium Laureth Sulfate, Decyl Glucoside, and Phenoxyethanol were then added and mixed. The fragrance was then added. The citric acid was then added and the ingredients mixed until the citric acid was completely dissolved. The pH was adjusted to between 6.4 and 7.2.

Example 5

The skin-contactable elements of Examples 1-3 were mounted on an apparatus for abrasively treating the skin using the adaptor described in Example 1. The motorized apparatus is commercially available from Neutrogena Corporation (Los Angeles, CA) as an applicator of a micro dermabrasion system under the name, "NEUTROGENA Advanced Solutions[TM] At Home MicroDeniiabrasion System." The speed setting used was the "high" setting.

A clinical assessment was performed in which 16 subjects performed a microdermabrasion treatment once per day in their homes. Each subject tested the systems described in Examples 1-3 on a particular spot on his or her forearm. At days 1, 5, and 10, the subjects were clinically evaluated for cell proliferation a manner similar to that described in US patent 5,456,260. "Fluorescence detection of cell proliferation," assigned to General Hospital incorporated herein by trans-epidermal water loss, moisurization, and hydration. The results are shown below

## MOISTURIZATION

## CELL PROLIFERATION

## TRANS-EPIDERMAL WATER LOSS

## HYDRATION

## Example 6

A nonwoven pad identified as 85 gsm spunbond and consisting of three layers (including 20% polyester, 80% rayon) having a pattern of raised dots having EVA binder coated on the dots; available from Green Bay Nonwovens as SX-247 was provided. This was cut into 41 mm diameter pads to which 0.75 grams of cleansing composition was applied thereon, in a manner similar to Example 1, to form a skin-contactable element.

## Example 7

The skin-contactable element of Example 4 and the skin-contactable element of Example 6 were tested for cell proliferation in a manner similar to that described in Example 5. The subjects were evaluated after 5 days of treatment. A comparison of the results shows a % increase in cell proliferation over baseline skin of about 52% for the pad of Example 4 as compared to about 33% for the pad of example 6. The pad of Example 4 showed superior cell proliferation versus the pad of comparative example 6.

Example 8

A skin-contactable element formed in the same manner as described in Example 4 , except that the nonwoven fibrous material had a basis weight of about 300gsm and the concentration of abrasive system applied was increased to 11%, yielding a basis weight of abrasive system associated with the pad of about 34 gsm (1 ounce per square yard).

Example 9

A skin-contactable element formed in the same manner as described in Example 4 , except that the nonwoven fibrous material had a basis weight of 400 gsm and the concentration of abrasive system applied was increased to 8.3%, yielding basis weight of abrasive system of about 34 gsm (1 ounce per square yard).

Example 10

The skin-contactable element of Example 8 and Example 9 were tested for cell proliferation similar to Example 7, except that treatments were performed on both the volar forearm and the face. The subjects were evaluated after 1 week and 2 weeks of treatments. On the face, the skin-contactable element of Example 8 provided greater cell proliferation (about 47%) at 2 weeks than the skin-contactable element of Example 9 (about 29% cell proliferation).

Example 11

A skin-contactable element similar to that described in Example 8, except that a concentration of abrasive system of about 17.7% was used, resulting in a abrasive system basis weight of about 1.6 ounces per square yard. The skin-contactable element was otherwise similar. The sample was analyzed for surface roughness using the Primos Skin Measuring Device described previously. The maximum surface roughness was about 758 microns. The average surface roughness was about 94.6 microns.

Comparative Example 12

A sample of OLAY Total Effects Daily Cleansing Treatments (available from Procter and Gamble of Cincinnati, OH) was analyzed for surface roughness using a similar manner as described above. The maximum surface roughness was about 180 microns. The average surface roughness was about 21microns.

Comparative Example 13 (C13)

SCOTCH BRITE pad # 86 was evaluated for Displacement, Compressibility, and Durable Abrasiveness. The results are provided in Table 1 below.

Comparative Example 14 (CI4)

SCOTCH BRITE pad # 96 was evaluated for Displacement, Compressibility, and Durable Abrasiveness. The results are provided in Table 1 below.

Comparative Example 15 (15)

BRILLO Scrub and Toss, commercially available from Church & Dwight of Princeton, New Jersey was evaluated for Displacement, Compressibility, and Durable Abrasiveness. The results are provided in Table 1 below.

Examples 16-18 (E16-E18)

Skin-contactable elements with varying basis weight of fiber and abrasive system were prepared. No cleansing formulation was applied to the pads. They were otherwise identical to the skin--contactable element described in Example 1. These were evaluated for Displacement, Compressibility, and Durable Abrasiveness. The results are provided in Table 1 below.

Examples 19-20 (E19-E20)

Skin-contactable elements with varying basis weight, of fiber and abrasive system were prepared. No cleansing formulation was applied to the pads. They were otherwise identical to the skin--contactable element described in Example 1. These were evaluated for Displacement, Compressibility, and Durable Abrasiveness. The results are provided in Table 1 below.

Example 21 (E2H

A skin contactable element was prepared similarly to Example 4, except that the basis weight of fiber was 300gsm pad and the basis weight of abrasive system was 1.6 osy. These were evaluated for Displacement, Compressibility, Durable Abrasiveness, and Plain Abrasiveness. The results are provided in Table 1 below.

Comparative Example 22

The skin contactable element of Example 6 was evaluated for Displacement, Compressibility, Durable Abrasiveness, and Plain Abrasiveness. The results are provided in Table 1 below.

Comparative Example 23

OLAY Total Effects Daily Cleansing Treatments (available from Procter and Gamble of Cincinnati, OH) was evaluated for Displacement, Compressibility, and Durable Abrasiveness. The results are reported in Table 1 below.

TABLE 1

| Ref. | Skin-Contactable Element, Identifier | Displacement (mm) | Compression (%) | Durable Abrasiveness | Plain Abrasiveness |
|------|--------------------------------------|-------------------|-----------------|----------------------|--------------------|
| C13 | SCOTCH-BRITE #86 | 4.0 | 28.5 | 33.3 | ---- |
| C14 | SCOTCH-BR1TE #96 | 2.3 | 20.7 | 14.3 | ---- |
| C15 | BRILLO SCRUB N TOSS | 0.43 | 12.3 | 20.8 | ---- |
| E16 | 200gsm pad / 25.5 gsm abrasive system | 0.41 | 15.0 | 3.1 | ---- |
| E17 | 300gsmpad/32gsm abrasive system | 0.52 | 11.9 | 7.8 | ---- |
| E18 | 400gsm pad / 32 gsm abrasive system | 0.26 | 6.9 | 4.0 | ---- |
| E19 | 300gsm pad / 13.6 gsm abrasive system | ---- | ---- | 3.7 | ---- |
| E20 | 300gsm pad / 20 gsm abrasive system | ---- | ---- | 5.6 | ---- |
| E21 | 300gsm pad / 54 gsm abrasive system | 0.36 | 8.8 | 4.9 | 1.2 |
| C22 | GREEN BAY | 0.21 | 16.1 | <1 | ---- |
| C23 | OLAY TOTAL EFFECTS | 0.12 | 20.0 | 1.7 | ---- |

Embodiments of the invention:

1. A method for mechanical skin resurfacing techniques comprising the steps of:

    a) contacting the skin with an apparatus comprising a motor and a skin-contactable element comprising a fibrous structure and an abrasive system bonded to such structure;
    b) imparting mechanical energy to the skin via the skin-contactable element;
    c) moving the skin-contactable element to contact a plurality of discrete areas on the skin.

2. The method of embodiment 1, wherein the abrasive system is chemically bonded to the fibrous structure

3. The method of embodiment 2, wherein the chemical bond is an adhesive bond.

4. The method of embodiment 1, wherein the abrasive system comprises a polymer having a glass transition temperature greater than about -20°C.

5. The method of embodiment 1, wherein the abrasive system is present in a weight ratio of abrasive system to fiber that is between about 5% and about 30%.

6. The method of embodiment 1, wherein the abrasive system comprises a plurality of discrete abrasive units distributed among the fibers of the fibrous structure.

7. The method of embodiment 6, wherein the discrete units have a maximum linear dimension between about 0.05mm to about 0.5mm.

8. The method of embodiment 1, wherein the abrasive system comprises a coating formed on the fibers.

9. The method of embodiment 1, wherein the fibrous structure comprise a nonwoven material.

10. The method of embodiment 9, wherein the nonwoven material comprises a needlepunched nonwoven material.

11. The method of embodiment 9, wherein the nonwoven material has a thickness from about 1mm to about 5mm.

12. A method for mechanical skin resurfacing techniques comprising the steps of:

    a) contacting skin with an apparatus comprising a motor and an abrasive skin-contactable element that comprises a fibrous structure;

    b) imparting mechanical energy to the skin via the skin-contactable element;

    c) moving the skin-contactable element to contact a plurality of discrete areas on the skin.

13. A disposable skin treatment element comprises a fibrous structure having

    a) a first major surface having associated therewith an abrasive system and
    b) a second major surface, generally opposite the first major surface, arranged and configured to reversibly engage a fastener of a motion-generating unit.

14. The element of embodiment 13, wherein the abrasive system comprises a plurality of discrete abrasive units bonded to fibers defining the first major surface.

15. The element of embodiment 14, wherein the abrasive units may include a polymer having a glass transition temperature greater than about -20°C.

16. The element of embodiment 13, wherein the first major surface of the fibrous structure is stiffer than the second major surface.

17. The element of embodiment 16, wherein the fibrous structure proximate the first major surface is stiffer than the more resilient fibrous structure disposed away from the first major surface.

18. The element of embodiment 17, wherein the resilient portion of the fibrous structure has a thickness from about 1mm to about 5mm.

19. A disposable skin treatment element comprising a fibrous structure having:

a) a first major surface having associated therewith an abrasive stiffening system and
b) a second major surface, generally opposite the first major surface, more resilient than the first major surface that is arranged and configured to reversibly engage a fastener of a motion-generating unit.

20. A disposable skin treatment element comprising a resilient structure having a thickness and comprising:

a) a first major surface having associated therewith an abrasive stiffening system and
b) a second major surface, generally opposite the first major surface, more resilient than the first major surface that is arranged and configured to reversibly engage a fastener of a motion-generating unit

wherein the abrasive stiffening system is disposed on and adjacent the first major surface and wherein the second major surface and adjacent more resilient portion combine to provide more than 50% of the thickness of the resilient structure.

21. A disposable skin treatment element for use with a handheld device, comprising a resilient structure having a Durable Abrasiveness from 2 to 14.

22. The element of embodiment 21, wherein said article has a Displacement from about 0.2 mm to about 2.0 mm.

23. The element of embodiment 22, wherein said article has a Displacement from about 0.25 mm to about 1.0 mm.

24. The element of embodiment 23, wherein said article has a Displacement from about 0.25 mm to about 0.8 mm.

25. The element of embodiment 21, wherein said article has a Compressibility of less than about 20%.

26. The element of embodiment 25, wherein said article has a Compressibility of less than about 15%.

27. The element of embodiment 26, wherein said article has a Compressibility from about 3% to about 13%.

28. The element of embodiment 21, wherein said article has a thickness from about 1.5 mm to about 4.5 mm.

29. The element of embodiment 21, wherein said article has a maximum surface roughness from about 200 microns to about 3000 microns.

30. The element of embodiment 29, wherein the article has a maximum surface roughness from about 350 microns to about 1500 microns.

31. The element of embodiment 21, wherein the article has an average surface roughness from about 25 microns to about 300 microns.

32. The element of embodiment 21, wherein the article has a Durable Abrasiveness from about 3 to about 10.

33. The element of embodiment 21, wherein the article, wherein the article comprises fibers.

34. The element of embodiment 33, wherein the article comprises needle-punched fibers.

35. The element of embodiment 33, wherein the article comprises fibers and an abrasive system bound to the fibers

36. A disposable skin treatment element for use with a handheld device, comprising a resilient structure having a Durable Abrasiveness between about 1 and about 14, and a Compressibility from about 7% to about 18%.

37. A disposable skin treatment element for use with a handheld device, comprising a resilient structure having a Durable Abrasiveness between about 1 and about 14, and a Displacement from about 0.15 to about 2.

38. A disposable skin treatment element for use with a handheld device, comprising a resilient structure having a Displacement from about 0.15 mm to about 2.0mm and a maximum surface roughness from about 350 microns to about 1500 microns.

39. A skin treatment element for use in mechanical skin resurfacing techniques comprising

    a) A first, skin-contacting surface;
    b) A second surface, substantially opposite the first, arranged and configured for releasable attachment to a handheld device;
    wherein the first surface of the pad has a Durable Abrasiveness from 2 to 14.

40. A system for mechanical skin resurfacing techniques comprising the steps of:

    a) a loop-engageable surface for reversibly engaging a fibrous skin-contactable element thereto;
    b) a motor; and
    c) means to transfer motion from the motor to the loop-engageable surface and to the fibrous skin-contactable element.

41. The system of embodiment 40, wherein said loop-engageable surface includes a plurality of protrusions for engaging said fibrous skin-contactable element.

42. The system of embodiment 40, wherein each of said protrusions is configured to have a surface area greater than about 0.002 mm2.

43. The system of embodiment 40, wherein each of said protrusions is configured to have a surface area greater than about 0.02 $mm^2$.

44. The system of embodiment 40, wherein each of said protrusions is configured to have a surface area from about 0.2 $mm^2$ to about 2 $mm^2$.

45. The system of embodiment 40, wherein said protrusions are rounded.

46. The system of embodiment 40, wherein said protrusions are present at an average number density that is greater than about 0.25 protrusions per square millimeter ($0.25/mm^2$)

47. The system of embodiment 46, wherein said protrusions are present at an average number density that is greater than about 0.75/ $mm^2$.

48. The system of embodiment 47, wherein said protrusions are present at an average number density that is greater than about 2/ $mm^2$.

49. The system of embodiment 40, wherein said protrusions are selected from a group consisting of curled, arcuate, mushroom-shaped, T-shaped, Y-shaped, and combinations thereof.

50. The system of embodiment 40, wherein said protrusions have a number density greater than about 0.25/ $mm^2$ and a height greater than about 0.05mm.

51. The system of embodiment 40, wherein said protrusions have a number density greater than about 0.75/ $mm^2$ and a height greater than about 0.10mm.

52. The system of embodiment 40, wherein said apparatus further comprises a body and said loop-engageable surface is adhesively secured to said body.

53. A coupling device for coupling a motorized apparatus to a disposable skin-contactable element, the coupling device comprising:

a) A first attachment surface arranged and configured for releasably coupling the coupling device to a surface of the motorized apparatus;

b) A second attachment surface arranged and configured for releasably coupling the disposable skin-contactable element to the coupling device;

wherein the first and second surfaces are coupled to transfer motion therebetween.

54. A coupling device for coupling a motorized apparatus to a disposable skin-contactable element, the coupling device comprising:

a) A first attachment portion arranged and configured for releasably coupling the coupling device to a surface of the motorized apparatus;

b) A second attachment portion arranged and configured for releasably coupling the disposable skin-contactable element to the coupling device;

wherein the first and second portions are coupled to transfer motion therebetween and the second attachment portion is arranged and configured for water-resistant coupling of the disposable skin-contactable element.

55. The coupling device of embodiment 54, wherein said water-resistant second attachment portion comprises a portion of a hook-and-loop attachment system.

56. The coupling device of embodiment 55, wherein said water-resistant second attachment portion comprises a loop-engageable structure.

57. The coupling device of embodiment 56, wherein the loop-engageable structure comprises a plurality of mushroom-shaped elements.

58. The coupling device of embodiment 54, wherein the first attachment portion is arranged and configured to provide a detachment strength to the motorized apparatus of about 0.5 pounds per square inch (psi) to about 20 psi.

59. The coupling device of embodiment 58, wherein the detachment strength is about 2 psi to about 10 psi.

60. The coupling device of embodiment 54, comprising a generally frusto-conical section between the first and second attachment portions.

61. The coupling device of embodiment 54, comprising a polymeric formed material.

62. The coupling device of embodiment 61, wherein the polymeric formed material comprises a thermoplastic structure.

63. The coupling device of embodiment 62, wherein the thermoplastic structure comprises an injection molded structure.

64. The coupling device of embodiment 62, wherein the thermoplastic structure comprises a vacuum-formed structure.

65. A system for abrasively treating an expanse of skin, comprising

a) A handheld, motorized apparatus arranged and configured to impart motion to skin placed in contact therewith;

b) A disposable, skin-contactable element; and

c) A coupling device comprising

i) A first attachment portion arranged and configured for releasably coupling the coupling device to a surface of the motorized apparatus; and

ii) A second attachment portion arranged and configured for releasably coupling the disposable skin-contactable element to the coupling device;

wherein the first and second portions are coupled to transfer motion therebetween and the second attachment portion is arranged and configured for water-resistant coupling of the disposable skin-contactable element.

**Claims**

1. A coupling device for coupling a motorized apparatus to a disposable skin-contactable element, the coupling device comprising:

   a. a first attachment surface arranged and configured for releasably coupling the coupling device to a surface of the motorized apparatus;
   b. a second attachment surface arranged and configured for releasably coupling the disposable skin-contactable element to the coupling device;

   wherein the first and second surfaces are coupled to transfer motion therebetween.

2. A coupling device according to claim 1, wherein the second attachment portion is arranged and configured for water-resistant coupling of the disposable skin-contactable element.

3. The coupling device of claim 2, wherein said water-resistant second attachment portion comprises a portion of a hook-and-loop attachment system, or a loop-engageable structure.

4. The coupling device of claim 3, wherein the loop-engageable structure comprises a plurality of mushroom-shaped elements.

5. The coupling device of claim 2, wherein the first attachment portion is arranged and configured to provide a detachment strength to the motorized apparatus of about 0.5 pounds per square inch (psi) to about 20 psi, or about 2 psi to about 10 psi.

6. The coupling device of claim 2, comprising a generally frusto-conical section between the first and second attachment portions.

7. The coupling device of claim 2, comprising a polymeric formed material.

8. The coupling device of claim 7, wherein the polymeric formed material comprises a thermoplastic structure.

9. The coupling device of claim 8, wherein the thermoplastic structure comprises an injection molded structure, or a vacuum-formed structure.

10. A system for abrasively treating an expanse of skin, comprising

    a. a handheld, motorized apparatus arranged and configured to impart motion to skin placed in contact therewith;
    b. a disposable, skin-contactable element; and
    c. the coupling device according to claim 2.

# FIG. 1

# FIG. 2

# FIG. 3A

27

37

33

31

39

35

# FIG. 3B

27

32

34b

34c

34d

34a

# FIG. 4A

# FIG. 5

# FIG. 4B

# FIG. 6

# FIG. 7

**FIG. 8**

**FIG. 9**

**FIG. 10**

# FIG. 11

# FIG. 12

EP 2 314 237 A1

# FIG. 13

# FIG. 14

33

Europäisches
Patentamt

European
Patent Office

Office européen
des brevets

# EUROPEAN SEARCH REPORT

Application Number

EP 10 19 6030

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 0 381 962 A (PETER BAUSCH GMBH & CO. KG.) 16 August 1990 (1990-08-16) | 1-3,10 | INV. A61B17/54 |
| Y | * abstract; figures * <br> * column 2, line 52 - column 3, line 22 * | 4-9 | |
| Y | US 5 785 784 A (CHESLEY ET AL.) 28 July 1998 (1998-07-28) * the whole document * | 4-9 | |
| A | FR 2 773 461 A (FAVIE) 16 July 1999 (1999-07-16) * abstract; figures 1-3 * * page 1, lines 5-24 * | 1 | |
| A | DE 299 17 099 U1 (LEIBETSEDER) 10 February 2000 (2000-02-10) * page 1, lines 3-8,23-32; figures * | 1 | |

| TECHNICAL FIELDS SEARCHED (IPC) |
|---|
| A61B <br> A45D <br> A43D |

The present search report has been drawn up for all claims

| Place of search | Date of completion of the search | Examiner |
|---|---|---|
| The Hague | 16 March 2011 | Giménez Burgos, R |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 10 19 6030

This annex lists the patent family members relating to the patent documents cited in the above-mentioned European search report.
The members are as contained in the European Patent Office EDP file on
The European Patent Office is in no way liable for these particulars which are merely given for the purpose of information.

16-03-2011

| Patent document cited in search report | | Publication date | Patent family member(s) | | Publication date |
|---|---|---|---|---|---|
| EP 0381962 | A | 16-08-1990 | AT<br>DE | 106703 T<br>3903828 A1 | 15-06-1994<br>16-08-1990 |
| US 5785784 | A | 28-07-1998 | NONE | | |
| FR 2773461 | A | 16-07-1999 | NONE | | |
| DE 29917099 | U1 | 10-02-2000 | NONE | | |

For more details about this annex : see Official Journal of the European Patent Office, No. 12/82

# REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20050148907 A
- WO 11023655 A
- US 11023655 B
- US 5456260 A

**Non-patent literature cited in the description**

- **CASSEL ; P. DIVITO.** Use of DSC To Obtain Accurate Thermodynamic and Kinetic Data. *American Laboratory,* January 1994, 14-19
- **B. WUNDERLICH.** Thermal Analysis. Academic Press, Inc, 1990